Europäisches Patentamt

European Patent Office

Office européen des brevets

(19)

(11) Veröffentlichungsnummer: **0 226 701**
**B1**

(12) **EUROPÄISCHE PATENTSCHRIFT**

(45) Veröffentlichungstag der Patentschrift:
13.06.90

(51) Int. Cl.⁵: **A61B 17/58**

(21) Anmeldenummer: 86109432.4

(22) Anmeldetag: **10.07.86**

(54) Knochennagel zur Versorgung von Oberarmfrakturen.

(30) Priorität: **06.12.85 DE 8534358 U**

(43) Veröffentlichungstag der Anmeldung:
**01.07.87 Patentblatt 87/27**

(45) Bekanntmachung des Hinweises auf die Patenterteilung:
**13.06.90 Patentblatt 90/24**

(84) Benannte Vertragsstaaten:
**CH DE FR GB IT LI**

(56) Entgegenhaltungen:
**DE-A- 2 359 644**
**FR-A- 2 064 915**
**FR-A- 2 387 637**
**US-A- 2 381 050**

(73) Patentinhaber: **Howmedica GmbH,**
**Professor-Küntscher-Str. 1-5, D-2314 Schönkirchen ü. Kiel(DE)**

(72) Erfinder: **Seidel, Hartmut Richard Alfred, Dr. med., Reventlowstrasse 48, D-2000 Hamburg 52(DE)**
Erfinder: **Harder, Hans Erich, Mecklenburger Strasse 35, D-2316 Probsteierhagen(DE)**
Erfinder: **Behrens, Klaus Friedrich Adolf, Dipl.-Ing., Am Sportplatz 8, D-2351 Rickling(DE)**

(74) Vertreter: **Dipl.-Ing. H. Hauck Dipl.-Phys. W. Schmitz Dipl.-Ing. E. Graalfs Dipl.-Ing. W. Wehnert Dr.-Ing. W. Döring, Neuer Wall 41, D-2000 Hamburg 36(DE)**

ACTORUM AG

## Beschreibung

Die Anmeldung betrifft einen Knochennagel zur Versorgung von Oberarmfrakturen mit einem im proximalen Bereich in die Markhöhle einführbaren hohlen Schaft, der am distalen Ende aufgeschlitzt ist, einem Spreizkörper, der mit einer Schraube im Inneren des Schaftes zusammenwirkt und der durch Drehen der Schraube in den Schaft hineingezogen wird und das aufgeschlitze Ende radial aufweitet, wobei das Querschnittsprofil des Schaftes in Umfangsrichtung zumindest an der Innenseite abwechselnd Erhebungen und Vertiefungen aufweist. Solch ein Knochennagel ist aus DE-A 2 359 644 bekannt.

In die Markhöhle einführbare Nägel zur Versorgung von Frakturen bei Röhrenknochen werden in den unterschiedlichsten Ausführungsformen und bei nahezu allen Röhrenknochen verwendet. Besonders bewährt haben sich sogenannte Verriegelungsnägel, die mit Hilfe von Querschrauben die Frakturfragmente statisch festlegen. Dadurch wird eine Verkürzung des Knochens infolge von Belastung während der ersten Heilungsphase vermieden. Außerdem wird mit Hilfe eines Verriegelungsnagels eine hohe Drehstabilität erzielt. Nach dem Entfernen der Knochenschrauben ist auch eine dynamische Belastung der Fraktur möglich, durch welche bekanntlich der Wachstumsprozeß im Frakturbereich stimuliert wird.

Im Prinzip bietet sich ein Verriegelungsnagel auch für die Versorgung von Oberarmbrüchen an. Durch die Knochenschrauben besteht jedoch die Gefahr der Verletzung von wichtigen Nervensträngen, so daß Verriegelungsnägel nur dann bei Oberarmbrüchen verwendet werden können, wenn die Frakturstelle in einem günstigen Bereich liegt.

Der Erfindung liegt daher die Aufgabe zugrunde, einen intramedullären Knochennagel zu schaffen, der besonders für die Versorgung von Oberarmbrüchen geeignet ist.

Diese Aufgabe wird erfindungsgemäß dadurch gelöst, daß außerhalb des geschlitzten Bereiches in die inneren Erhebungen ein Innengewinde geformt ist und der Spreizkörper vom Kopf einer Spreizschraube gebildet ist, deren Schaft einen Gewindeabschnitt aufweist, der mit dem Innengewinde zusammenwirkt.

Die günstigste Einführung eines Oberarmknochennagels liegt im proximalen Bereich. Eine Verankerung des erfindungsgemäßen Knochennagels erfolgt im distalen Bereich durch eine Verdübelung. Die Ausbildung eines Knochennagels an einem Ende als Spreizdübel ist grundsätzlich bekannt (EP 0 023 228). Der Innendurchmesser am distalen Ende ist beim bekannten Knochennagel in Richtung nach proximal stetig verkleinert durch eine entsprechende Verdickung der Wandung des hohlen Nagelschaftes. Mit diesem Innenkonus wirkt ein konischer Spreizkörper zusammen, der ein Innengewinde hat, in das eine Schraube eingeschraubt ist. Der Kopf der Schraube legt sich gegen eine Führung im Inneren des Nagelschaftes an. Das Werkzeug zur Betätigung der Schraube wird vom anderen Ende des Schaftes eingeführt. Dadurch kann der Spreizkörper in den Nagelschaft hineingezogen werden und spreizt dabei den geschlitzten Bereich des Schaftes radial nach außen, um ihn in Eingriff mit der Knochenwandung zu bringen.

Der bekannte Nagel weist einige Nachteile auf. Die Anbringung einer Führung im Inneren des Nagels und die Ausbildung der Verdickung im distalen Bereich ist fertigungstechnisch sehr aufwendig. Der maximale Durchmesser des Spreizkörpers darf den Außendurchmesser des Nagels nicht übersteigen, da andernfalls das Einführen des Knochennagels Probleme bereitet. Daher ist die maximal zur Verfügung stehende Durchmesseränderung zwangsläufig begrenzt. Der Verstellweg des Spreizkörpers zur Erzielung der maximalen Spreizung ist relativ kurz. Dementsprechend ist die überhaupt maximal erzielbare Spreizung relativ gering. Wird bei dem bekannten Nagel der Engquerschnitt vom Spreizkörper überschritten, wird die Befestigung im Knochenkanal wieder aufgehoben. Es muß daher eine axiale Begrenzung für den Spreizkörper vorgesehen werden. Beim Lösen der Dübelverbindung wird die Schraube in die entgegengesetzte Richtung gedreht. Es kann jedoch zwischen Nagelwandung und Spreizkörper ein Verklemmen stattfinden. Es kann daher geschehen, daß die Schraube vollständig herausgedreht wird, ohne daß der Spreizkörper sich bewegt hat. Es muß daher in einem solchen Falle auf den Kopf der Schraube aufgeschlagen werden, um die Dübelwirkung aufzuheben. Dabei muß Sorge dafür getragen werden, daß die Schraube mit dem Spreizkörper verbunden bleibt, andernfalls der Spreizkörper beim Entfernen des Nagels in der Markhöhle verbleibt.

Bei dem erfindungsgemäßen Knochennagel ist der Spreizkörper vom Kopf einer Schraube gebildet, die in ein Innengewinde des Nagelschaftes eingeschraubt wird. Dieses Innengewinde wird in nach innen stehende Vorsprünge des Nagelschaftes geschnitten. Zu diesem Zweck hat der Querschnitt des Nagels einen Innendurchmesser, der sich über seinen Umfang ändert, so daß Vertiefungen und Erhebungen entstehen. Besonders vorteilhaft ist die Ausbildung eines sogenannten Kleeblattprofils, wie es an sich durch sogenannte Küntschernägel bekanntgeworden ist. Der erfindungsgemäße Nagel hat über den größten Teil seiner Länge ein derartiges Profil, ohne jedoch wie beim Küntschernagel eine Längsschlitz aufzuweisen. Ein Knochennagel mit einem derartigen Querschnittsprofil sichert die Frakturfragmente wirksamer gegen Rotation als dies bei im Querschnitt kreisförmigen Nägeln der Fall ist.

Im Dübelbereich hat das erwähnte Querschnittprofil des erfindungsgemäßen Knochennagels den Vorteil, daß die Erhebungen zugleich Angriffspunkte für den Spreizkopf der Spreizschraube darstellen. Nach einer Ausgestaltung der Erfindung ist der durch die Erhebungen definierte minimale Radius im Inneren des Nagelschaftes zum distalen Ende hin größer als weiter einwärts. Auf diese Weise kann über die gesamte Länge des Spreizbereichs, welcher durch die Länge der achsparallelen Schlitze bestimmt ist, eine Aufspreizung durch den Spreizkopf der Schraube vorgenommen werden. Es versteht sich, daß das Ende des Schaftes der Spreizschraube Angriffsmittel aufweisen muß für ein Werkzeug zum Verdrehen der Schraube. Dieses Angriffsmittel kann nach einer Ausgestaltung der Neuerung durch einen Innenmehrkant gebildet werden, beispielsweise einen Innenvierkant.

Wie schon erwähnt, wird durch das besondere Querschnittsprofil der erfindungsgemäßen Kno-chenschraube nicht nur die Drehsicherheit für die Fraktursegmente erhalten, sondern zugleich ein maximaler Aufspreizgrad, welcher durch das Ausmaß der Erhebungen im Inneren des Nagelschaftes bestimmt ist. Der maximale Außendurchmesser bei einem Nagelschaft mit Kleeblattprofil wird hingegen beibehalten. Das Lösen der Spreizschraube vom Nagelschaft ist unproblematisch, da sich die Schraube am Innengewinde abstützt. Damit die Schraube jedoch nicht ungewollt herausgedreht wird, sieht eine Ausgestaltung der Erfindung vor, daß die Spreizschraube am Schaftende eine Durchmessererweiterung aufweist, im Schaft zwei quer zur Längsachse ausgerichtete Löcher zur Aufnahme eines Sicherungsstiftes geformt und so angeordnet sind, daß der Sicherungsstift mit der Durchmessererweiterung in Eingriff gelangt, wenn die Schraube aus dem Schaft herausgedreht wird. Die Lage des Sicherungsstiftes bzw. die Länge der Schraube sind so gewählt, daß ein vollständiges Lösen des Kopfes der Schraube aus dem Nagelschaft gewährleistet sein muß, bevor die Durchmessererweiterung mit dem Sicherungsstift in Eingriff gelangt. Durch den Anschlag der Durchmessererweiterung mit dem Sicherungsstift spürt der Chirurg, daß dieser Punkt erreicht ist.

Eine andere Ausgestaltung der Erfindung sieht vor, daß im geringen Abstand zum proximalen Ende des Schaftes zwei quer zur Schaftlängsachse ausgerichtete Löcher zur Aufnahme einer Knochenschraube geformt sind. Mit Hilfe einer Knochenschraube kann der erfindungsgemäße Nagel an einem Ende als Verriegelungsnagel wirken, während das andere Ende spreizdübelartig festgelegt wird. Auf diese Weise kann eine statische Abstützung des Knochens erfolgen und verhindert werden, daß eine Verkürzung während der ersten Heilungsphase eintritt. Die Länge des erfindungsgemäßen Nagels kann im übrigen an die Lage der Fraktur angepaßt werden. Der Knochennagel braucht nur so lang zu sein, daß die Frakturstelle ausreichend überbrückt wird. Bei nahe dem Oberarmgelenk liegenden Frakturen ist daher nur ein relativ kurzer Knochennagel erforderlich.

Eine weitere Ausgestaltung der Erfindung sieht vor, daß der proximale Bereich des Schaftes um einen kleinen Winkel abgebogen ist und die Schaftwand ein Langloch aufweist in dem Bereich, in dem die Achse des geraden Schaftabschnitts die Wand des abgebogenen Abschnitts durchstößt. Mit abgebogenem Abschnitt kann das Werkzeug zum Verdrehen der Spreizschraube nicht mehr durch den Hohlraum des Nagelschaftes hindurchgeführt werden. Die Abbiegung ist jedoch aus anatomischen Gründen vorteilhaft, damit das proximale Ende des Nagels im Hinblick auf die anatomische Ausbildung des Oberarmgelenks günstig positioniert werden kann. Dementsprechend weist der abgebogene Abschnitt ein Langloch auf, über das das Werkzeug eingeführt werden kann. Es ist daher in den Oberarmkopf ein zusätzliches Loch zu bohren, damit der lange und dünne Schaft des Werkzeugs über das Langloch in den Knochennagel eingeführt werden kann. Dies stellt jedoch weder für den Operateur noch für den Patienten ein zusätzliches Problem dar.

Eine andere Ausgestaltung der Erfindung sieht vor, daß in das proximale Ende des Schaftes zwei diametral gegenüberliegende Ausnehmungen geformt sind. Durch Eingriff eines Werkzeugs in die beiden Ausnehmungen kann der Nagel während und nach dem Einführen gedreht werden, um ihm bezüglich der Abwinklung die günstigste Drehlage zu erteilen bzw. um eine Rotation des distalen Fraktursegments vorzunehmen zur Ausrichtung der Fraktursegmente in die anatomisch richtige Drehlage.

Das proximale Schaftende kann nach einer weiteren Ausgestaltung der Erfindung ein Innengewinde aufweisen. Das Innengewinde dient zum Anbringen eines Ein- und Ausschlagwerkzeugs. Ferner kann es zur Anbringung einer Ziellehre dienen beim Einsatz des erfindungsgemäßen Knochennagels als Verriegelungsnagel. In diesem Zusammenhang sieht eine weitere Ausgestaltung der Erfindung vor, daß auf der proximalen Seite des Innengewindes ein Innenkonus geformt ist, der sich nach proximal erweitert. Ein- und Ausschlaggerät können entsprechende Außenkonen haben. Die Ziellehre kann ebenfalls mit einem Außenkonus versehen werden, um die Axiallage des Zielgeräts einzustellen.

Frakturen des Oberarms finden auch unmittelbar im Gelenkkopf statt. Um auch hier eine wirksame Versorgung zu erhalten, sieht eine Ausgestaltung der Erfindung vor, daß mit dem proximalen Ende des Schafts eine Klammer verbunden ist, die spinnenartig angeordnete, biegbare und etwas nach unten ab-gebogene Arme aufweist. Gerichtete Frakturseg-mente können von den zugeordneten Armen aufgefangen und abgestützt werden. Durch entsprechendes Verbiegen und Auf-Länge-Schneiden der Arme kann eine individuelle Anpassung an den jeweiligen Vorfall stattfinden. Nicht benötige Arme können vom Operateur mit Hilfe einer Zange oder dergleichen entfernt werden. Es versteht sich, daß ein derartiges Osteosynthesehilfsmittel auch mit einem Spreizdübel verwendet werden kann, der in herkömmlicher Weise ausgebildet ist.

Die Arme sind nach Möglichkeit in gleichen Umfangsabständen angeordnet. Es sind jedoch nicht über den gesamten Umfangsbereich Arme erforderlich. Es reicht aus, wenn etwa ein Bereich von 200° durch einzelne Arme abgedeckt ist. Um die Anpassung der Klammer bzw. der Arme an die jeweilige Fraktur zu optimieren, ist nach einer weiteren Ausgestaltung der Erfindung die Drehlage der Klammer wählbar. Eine beliebige Einstellung der Drehlage wird nach einer weiteren Ausgestaltung der Erfindung dadurch erreicht, daß die Klammer mittig einen axialen konischen Zapfen aufweist, der mit einem Innenkonus des Schaftes zusammenwirkt, der Zapfen eine axiale Durchbohrung aufweist für eine Befestigungsschraube, deren Kopf sich gegen die Außenseite der Klammer legt und deren Schaft mit einem Innengewinde des Lagerschaftes zusammenwirkt. Der Kopf der Befestigungsschraube ist vorzugsweise in der Klammer abgesenkt und mit einer äußeren Rundung versehen, deren Radius annähernd mit dem Radius der Außenseite der Arme übereinstimmt.

Die Erfindung wird nachfolgend anhand von Zeichnungen näher erläutert.

Fig. 1 zeigt teilweise im Schnitt einen Oberarmknochennagel nach der Erfindung.

Fig. 2 zeigt einen Schnitt durch den Nagel nach Fig. 1 entlang der Linie 2-2.

Fig. 3 zeigt vergrößert den distalen Bereich des Nagels nach Fig. 1.

Fig. 4 zeigt in Seitenansicht teilweise im Schnitt den proximalen Nagelbereich des Nagels nach Fig. 1.

Fig. 5 zeigt einen Schnitt durch die Darstellung nach Fig. 2 entlang der Linie 5-5.

Fig. 6 zeigt eine Seitenansicht des proximalen Endbereichs des Nagels nach Fig. 4.

Fig. 7 zeigt eine Seitenansicht der Spreizschraube des Knochennagels nach Fig. 1.

Fig. 8 zeigt eine Draufsicht auf eine Klammer für den Knochennagel nach Fig. 1.

Fig. 9 zeigt einen Schnitt durch den Lagerkörper nach Fig. 8.

Fig. 10 zeigt eine Seitenansicht der Befestigungsschraube für die Klammer nach den Figuren 8 und 9.

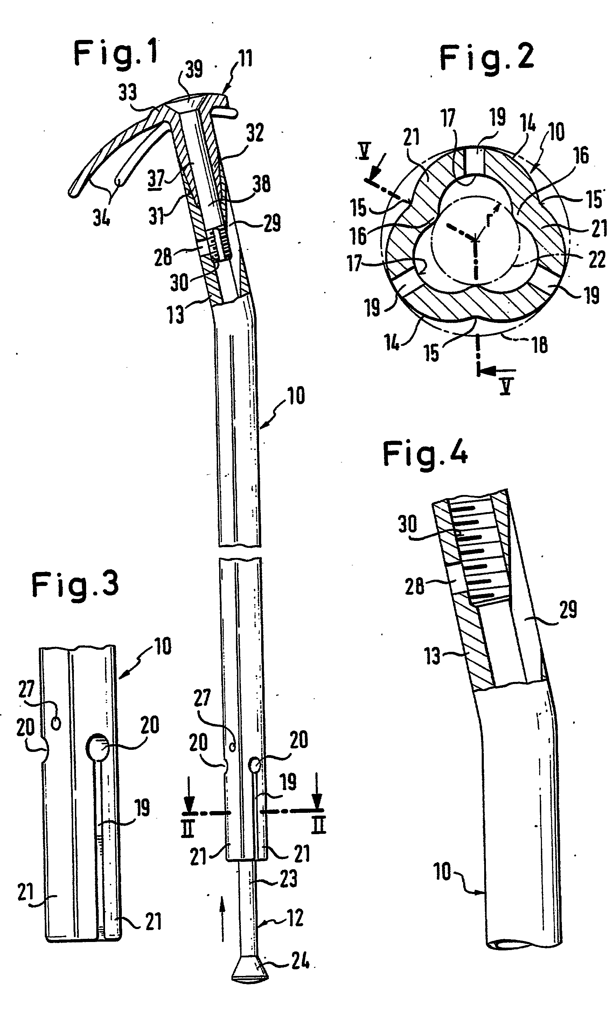

Der in Fig. 1 gezeigte Oberarm-Knochennagel weist einen Schaft 10 auf, an dessen proximalem Ende eine Klammer 11 angebracht ist und der am distalen Ende mit einer Spreizschraube 12 zusammenwirkt. Der Schaft 10 besitzt im oberen Viertel einen Abschnitt 13, der um einen kleinen Winkel gegenüber der Achse des übrigen Abschnitts abgebogen ist. Der Querschnitt des Abschnitts 13 ist kreisförmig, und zwar innen und außen. Der Querschnitt des übrigen Schaftes 10 geht aus Fig. 2 hervor. Das Querschnittsprofil ist in Form eines dreiblättrigen Kleeblatts ausgebildet mit drei bogenförmigen Erhebungen 14, die in gleichem Umfangsabstand von 120° angeordnet sind, wobei sich der Bogen einer Erhebung 14 um etwa 180° erstreckt. Zwischen den Erhebungen sind Vertiefungen 15 oder Nuten geformt. Die Erhebungen 14 gehen über abgerundete Bereiche in die Vertiefungen 15 über. Die Wanddicke des Schaftes ist über den Umfang gleich, so daß sich innen ein entsprechendes Profil ergibt mit inneren Erhebungen 16 und inneren Vertiefungen 17. Die Fertigung des gezeigten Knochennagels erfolgt von einem im Querschnitt kreisförmigen Rohling aus, der durch Kaltverformung in das in Fig. 2 gezeigte Querschnittsprofil gebracht wird. Der Außendurchmesser des Rohlings ist durch den strichpunktiert gezeichneten Kreis 18 angedeutet. Man erkennt, daß im Bereich der Erhebungen 14 der Außendurchmesser des Rohlings beibehalten ist.

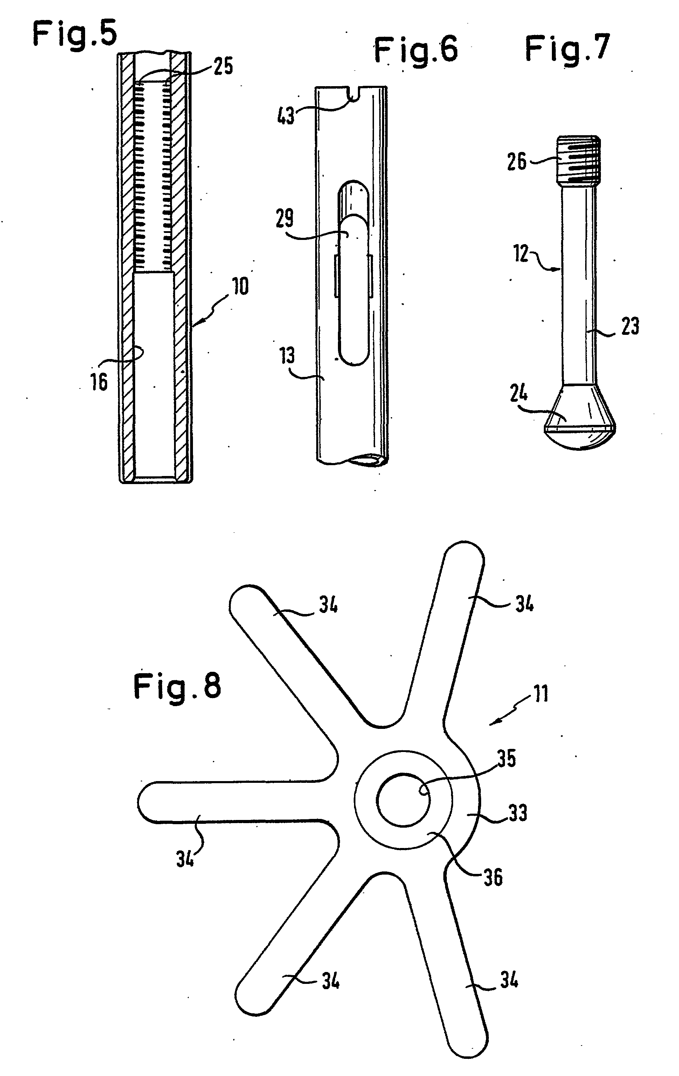

Im distalen Endbereich des Schaftes 10 sind drei achsparallele Schlitze 19 geformt. Wie aus Fig. 2 zu erkennen, liegen sie im Bereich des Maximums der Erhebungen 14. Sie enden in Löchern 20, deren Durchmesser größer ist als die Breite der Schlitze 19. Auf diese Weise sind drei Lamellen 21 geschaffen, deren Anbindung an den Schaft 10 durch die Löcher 20 einen Engquerschnitt erhält. Im Bereich der Löcher 20 ist mithin eine Art federndes Gelenk gebildet. Auf der dem distalen Ende abgewandten Seite der Löcher 20 ist im Inneren des Schaftes 10 ein Gewindeabschnitt geformt. Der Gewindeabschnitt ist ausschließlich in den Erhebungen 16 geformt, deren Radius durch den strichpunktierten Kreis 22 angedeutet ist. Dieser Radius r ändert sich im übrigen im Bereich der Schlitze 19, und zwar derart, daß er am distalen Ende am größten und unmittelbar vor den Löchern 20 am kleinsten ist. Dies wird zum Beispiel durch ein entsprechendes konisches Aufbohren des rohrförmigen Rohlings erreicht. Der erwähnte Gewindeabschnitt dient zur Aufnahme des Schaftes 23 der Spreizschraube 12. In Fig. 5 ist das Gewinde mit 25 bezeichnet. In Fig. 7 ist die Spreizschraube 12 vergrößert dargestellt. Nur das Schaftende besitzt einen Gewindeabschnitt 26, der einen größeren Durchmesser hat als der übrige Schaft 23. Im Nagelschaft 10 sind zwei in Querrichtung ausgerichtete Löcher geformt, von denen eines bei 27 gezeigt ist. Ein in die Löcher 27 eingesteckter Sicherungsstift verhindert, daß die Schraube 12 zu weit aus dem Lagerschaft 10 herausgeschraubt wird.

Im abgewinkelten Abschnitt 13 des Schaftes 10 ist eine Querbohrung 28 geformt. Diametral gegenüber der Querbohrung liegt ein Langloch 29 (siehe auch Fig. 6). Das Langloch ist in Verlängerung der Längsachse des unteren Teils des Schaftes 10 in der Wandung des abgewinkelten Abschnitts 13 geformt. Durch das Langloch 29 wird der dünne lange Schaft eines Werkzeugs hindurch in den Schaft 10 eingeführt, um die Spreizschraube 12 zu drehen. Zu diesem Zweck hat die Spreizschraube 12, was in den Figuren nicht dargestellt ist, am Ende seines Schaftes 23 einen Vier- ode Sechskant. Durch Bohrung 28 und Schlitz 29 kann quer eine Knochenschraube hindurchgeschraubt werden, um den Schaft 10 im entsprechenden Fraktursegment axial festzulegen.

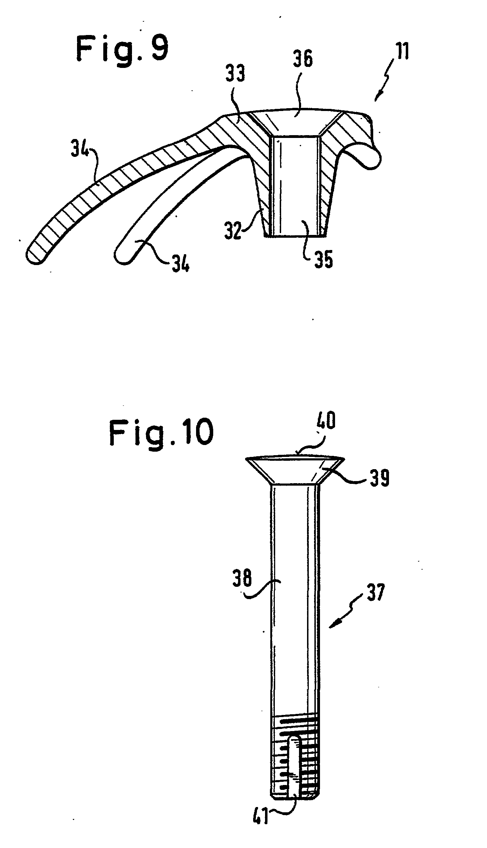

Der Abschnitt 13 weist ein Innengewinde 30 auf, das bis zu einem Innenkonus 31 (siehe (Fig. 1) am proximalen Ende des Schaftes 10 reicht. Mit dem Innenkonus 31, dessen Durchmesser noch proximal größer wird, wirkt ein konischer Zapfen 32 der Klammer 11 zusammen. Der Innenkonus 31 kann jedoch auch mit einem entsprechenden Außenkonus eines Ein- und Ausschlagwerkzeugs zusammenwirken sowie mit einer Ziellehre zum Anbringen einer Knochenschraube, worauf weiter unten noch näher eingegangen wird. Die Klammer 11 geht näher aus den Figuren 8 und 9 hervor. Bei der Ausführungsform nach Fig. 1 muß der Nagel versenkt eingeschlagen werden, was den Vorteil aufweist, daß nach gewissem Heilungsverlauf und sogenannter Dynamisierung, bei der die Klammer 11 und eine eventuelle Querschraube entfernt werden, kein Metall mehr die Gelenkmobilität einschränkt.

Am oberen Ende des Zapfens 32 ist ein radialer, ringförmiger Flansch 33 angeformt, an den in gleichen Abständen fünf Arme 34 angeformt sind. Die Arme beschreiben einen Bereich von annähernd 200°, während im übrigen Bereich Arme nicht vorhanden sind. Die spinnenförmigen Arme 34 sind, was in Fig. 9 zu erkennen ist, kreisförmig nach unten gebogen und umgeben den Zapfen 32 bzw. 32' korbartig. Sie sind ausreichend biegsam, um je nach Bedarf nach oben oder unten gebogen zu werden. Sie können außerdem mit Hilfe einer Zange oder dergleichen verkürzt oder völlig abgeschnitten werden. Der Flansch 33 ist an der Oberseite gerundet (siehe Fig. 9), wobei der Radius dieser Rundung annähernd mit dem Radius der Arme 34 übereinstimmt. Der Zapfen 32 weist eine Durchbohrung 35 auf, die sich nach oben bei 36 konisch erweitert. Durch die Durchbohrung 35 wird eine Befestigungsschraube 37 hindurchgeführt, die in Fig. 10 vergrößert dargestellt ist. Der Gewindeschaft 38 der Befestigungsschraube 37 wird in das Innengewinde 30 (Fig. 4) des Schaftes 10 geschraubt. Der Kopf 39 der Schraube 37 ist konisch und wird von dem Innenkonus 36 der Klammer 11 aufgenommen. Die Außenseite der Schraube ist bei 40 gerundet, wobei diese Rundung mit der Rundung an der Oberseite des Ringflansches 33 übereinstimmt (siehe auch Fig. 1). Am Ende des Schaftes 38 ist ein an sich bekanntes Sicherungselement 41 aus Kunststoff angebracht, um ein ungewolltes Lösen der Befestigungsschraube 37 zu vermeiden.

Die Wirkungsweise des beschriebenen Knochennagels ist wie folgt. Nachdem im Oberarmknochen im proximalen Endbereich ein Einführloch gebohrt oder auf andere Art eingebracht worden ist, wird der Schaft 10 mit aufgenommener Spreizschraube 24 ohne Klammer 11 in den Knochenkanal eingetrieben. Der Durchmesser des Kopfes 24 der Spreizschraube 12 ist nicht größer als der Außendurchmesser des Schaftes 10. Das Einschlagwerkzeug kann einen Konus aufweisen, der mit dem Innenkonus 31 am proximalen Endbereich des Schaftes 10 zusammenwirkt. Wie aus Fig. 6 zu erkennen, besitzt das proximale Ende zwei diametral gegenüberliegende schlitzartige Ausnehmungen 43. Das entsprechend ausgebildete Einschlagwerkzeug faßt in die Ausnehmungen 43, so daß der Schaft 10 um seine Achse gedreht werden kann. Dies geschieht zum Zwecke der Reponierung der Fraktursegmente. Ist der Schaft 10 vollständig eingeschlagen, wird ein weiteres Loch für ein Werkzeug zum Betätigen der Spreizschraube 12 gebohrt. Dieses Werkzeug enthält einen dünnen langen Schaft, der durch das Langloch 29 in den Schaft 10 eingeführt wird und mit dem Innenmehrkant des Schaftes 23 der Spreizschraube 12 zusammenwirkt. Die Schraube 12 wird in den Schaft 10 hineingedreht, wobei ihr Kopf 24 durch Eingriff an den Erhebungen 16 die Lamellen 21 radial nach außen spreizt in Eingriff mit der Knochenwandung, bis ein Festsitz im Knochen erzielt wird. Mit Hilfe einer durch die Bohrungen 28 und 29 hindurchgeführten Knochenschraube kann der Schaft 10 im oberen Bereich des Oberarmknochens axial festgelegt werden. Eine Verkürzung des Knochens wird dadurch vermieden und die Rotationsstabilität erhöht. Das Zielgerät zur Ermittlung der Bohrungen 28, 29 kann ebenfalls einen Konus aufweisen, der mit dem Innenkonus 31 des Schaftes 10 sowie den schlitzartigen Ausnehmungen 43 zusammenwirkt. Die Knochenschraube und Klammer kann nach einiger Zeit entfernt werden, nachdem eine ausreichende Kallusbildung an der Bruchstelle stattgefunden hat. Danach wirkt der Nagel als dynamisches Osteosynthesehilfsmittel, indem es eine Wechselbelastung des Knochens zuläßt. Diese fördert bekanntlich das Knochenwachstum. Zu diesem Zeitpunkt oder auch schon vorher kann die Klammer 11 angebracht werden. Die Drehlage entspricht den Frakturen im Gelenkkopfbereich. Entsprechend werden die Arme 34 gebogen, entfernt und/oder verkürzt. Mit Hilfe der Befestigungsschraube 37 wird die Klammer 11 fest mit dem Schaft 10 verbunden. Wird die Klammer 11 bei der ersten Versorgung eingesetzt, findet auch eine gewisse Kompression zwischen dem Dübel und der Klammer statt, was in manchen Versorgungsfällen ebenfalls erwünscht ist. Die Schraube 27 ist selbstgesichert, so daß sie sich nicht von allein lockert.

Soll der Knochennagel entfernt werden, wird, falls angewendet und nicht vorher geschehen, zunächst die Klammer 11 abgenommen. Anschließend wird mit Hilfe des bereits erwähnten Werkzeugs die Spreizschraube 12 aus dem Schaft 10 herausgeschraubt. Der in den Löchern 27 steckende Sicherungsstift wird von dem Gewindeabschnitt 26 der Schraube 12 erfaßt, so daß der Operateur feststellt, wann der Spreizkörper 24 ausreichend weit aus dem Schaft 10 herausbewegt worden ist. Außerdem verhindert der Sicherungsstift, daß die Spreizschraube 12 vollständig herausgedreht wird und dadurch im Knochenkanal verbleibt, wenn der Nagel entfernt wird.

Wie bereits erwähnt, wird der Schaft 10 aus einem geraden rohrförmigen Rohling geformt. Da im abgewinkelten Abschnitt 13 ein Innengewinde zu formen ist, wird der Rohling im übrigen Teil auf einen größeren Durchmesser aufgebohrt. Während dieses Fertigungsganges erfolgt auch das Einschneiden des Innengewindes 30 und die Ausformung des Innenkegels 31. Anschließend wird der gerade Teil des Schaftes 10 durch Kaltverformung in ein Profil gemäß Fig. 2 gebracht. Danach werden die Schlitze 19 geformt und das Innengewinde in die inneren Erhebungen 16 geschnitten. Durch die beschriebene Herstellungsweise ist der Knochennagel annähernd gratfrei und besitzt daher eine hohe Standfestigkeit.

**Patentansprüche**

1. Knochennagel zur Versorgung von Oberarmfrakturen mit einem im proximalen Bereich in die Markhöhle einführbaren hohlen Schaft (10), der am distalen Ende mehrere achsparallele Schlitze (19) aufweist, einem Spreizkörper (24), der mit einer Schraube im Inneren des Schaftes (10) zusammenwirkt und durch Drehen der Schraube in den Schaft (10) hineinziehbar ist und das aufgeschlitzte Ende radial aufweitet, wobei das Querschnittsprofil des Schaftes (10) in Umfangsrichtung zumindest an der Innenseite abwechselnd Erhebungen (16) und Vertiefungen (17) aufweist, dadurch gekennzeichnet, daß außerhalb des geschlitzten Bereiches (19, 20) in die inneren Erhebungen (16) ein Innengewinde (25) geformt ist und der Spreizkörper vom Kopf (24) einer Spreizschraube (12) gebildet ist, deren Schaft (23) einen Gewindeabschnitt (26) aufweist, der mit dem Innengewinde (25) zusammenwirkt.

2. Knochennagel nach Anspruch 1, dadurch gekennzeichnet, daß die achsparallelen Schlitze (19) im Bereich der Vertiefungen (17) liegen.

3. Knochennagel nach Anspruch 1 oder 2, dadurch gekennzeichnet, daß die Schlitze (19) proximal in einem Loch (20) enden, dessen Durchmesser größer als die Breite des Schlitzes (19) ist.

4. Knochennagel nach einem der Ansprüche 1 bis 3, dadurch gekennzeichnet, daß das Querschnittsprofil annähernd die Form eines dreiblättrigen Kleeblatts aufweist.

5. Knochennagel nach einem der Ansprüche 1 bis 4, dadurch gekennzeichnet, daß der durch die inneren Erhebungen (16) definierte Innenradius im geschlitzten Bereich vom distalen Ende nach proximal stetig abnimmt.

6. Knochennagel nach einem der Ansprüche 1 bis 5, dadurch gekennzeichnet, daß die Spreizschraube (12) am Schaftende eine Durchmessererweiterung (26) aufweist, im Schaft (10) quer zur Längsachse ausgerichtete Löcher (27) zur Aufnahme eines Sicherungsstiftes geformt und so angeordnet sind, daß der Spreizstift (12) mit der Durchmessererweiterung (26) in Eingriff gelangt, wenn die Schraube aus dem Schaft (10) herausgeschraubt wird.

7. Knochennagel nach einem der Ansprüche 1 bis 6, dadurch gekennzeichnet, daß das Ende des Schraubenschafts (23) einen Innenmehrkant aufweist zur Aufnahme des Mehrkantendes eines Schraubendrehers.

8. Knochennagel nach einem der Ansprüche 1 bis 7, dadurch gekennzeichnet, daß in geringem Abstand zum proximalen Ende des Nagelschafts (10) zwei quer zur Schaftlängsachse ausgerichtete Löcher (28, 29) zur Aufnahme einer Knochenschraube geformt sind.

9. Knochennagel nach einem der Ansprüche 1 bis 8, dadurch gekennzeichnet, daß im proximalen Bereich des Nagelschafts (10) ein Abschnitt (10) im kleinen Winkel abgebogen ist und die Schaftwand im Abschnitt (13) ein Langloch (29) aufweist in dem Bereich, in dem die Achse des geraden Schaftteils die Wand des abgebogenen Abschnitts (13) durchstößt.

10. Knochennagel nach einem der Ansprüche 1 bis 9, dadurch gekennzeichnet, daß in das proximale Ende des Nagelschafts (10) zwei diametral gegenüberliegende Ausnehmungen geformt sind.

11. Knochennagel nach einem der Ansprüche 1 bis 10, dadurch gekennzeichnet, daß in geringem Abstand zum proximalen Schaftende ein Innengewinde (30) geformt ist.

12. Knochennagel nach Anspruch 11, dadurch gekennzeichnet, daß auf der proximalen Seite des Innengewindes (30) ein Innenkonus (31) geformt ist, der sich nach proximal erweitert.

13. Knochennagel nach einem der Ansprüche 1 bis 12 , dadurch gekennzeichnet, daß mit dem proximalen Ende des Lagerschafts (10) eine Klammer (11) verbindbar ist, die spinnenartig angeordnete biegbare und etwas nach unten abgebogene Arme (34) aufweist.

14. Knochennagel nach Anspruch 13, dadurch gekennzeichnet, daß die Arme (34) in gleichmäßigen Umfangsabständen angeordnet sind, wobei jedoch nur in einem Umfangsbereich von etwa 200° Arme (34) angeordnet sind.

15. Knochennagel nach Anspruch 13 oder 14, dadurch gekennzeichnet, daß die Drehlage der Klammer (11) wählbar ist.

16. Knochennagel nach einem der Ansprüche 13 bis 15, dadurch gekennzeichnet, daß die Klammer (11) mittig einen axialen konischen Zapfen (32) aufweist, der mit einem Innenkonus (31) des Nagelschafts (10) zusammenwirkt, der Zapfen (32) eine axiale Durchbohrung (35) aufweist für eine Befestigungsschraube (37), deren Kopf (39) sich gegen die Außenseite der Klammer (11) legt und deren Schaft (38) mit einem Innengewinde (30) des Lagerschafts (10) zusammenwirkt.

17. Knochennagel nach Anspruch 16, dadurch gekennzeichnet, daß der Kopf (39) der Befestigungsschraube (37) gerundet ist und die Rundung an den Außenradius der gebogenen Arme (34) angepaßt ist.

**Claims**

1. An intermedullary pin for the treatment of upper arm fractures comprising a hollow shaft (10) insertable into the intermedullary cavity in the proximal area thereof, the shaft comprising at the distal end thereof a plurality of slots (19) running parallel to the axis, a spreading body (24) co-operating with a screwbolt in the interior of the shaft (10) and adapted to be pulled into the shaft (10) by rotating the screwbolt and radially expanding the slotted end, the cross-sectional profile of the shaft circumferentially comprising in turn elevations (16) and indentations (17) at least at the inner side, characterized in that outside the slotted area (19, 20) an internal thread (25) is formed in the internal elevations (16) and the spreading body is formed by the head (24) of a spreading screwbolt (12), the shaft (23) thereof comprising a threaded portion (26) co-operating with the internal thread (25).

2. Intemedullary pin as claimed in claim 1, characterized in that the slots (19) running parallel with the axis are positioned in the area of the indentations (17).

3. Intemedullary pin as claimed in the claims 1 or 2, characterized in that the slots (19) terminate proximally in an aperture (20), the diameter of which is larger than the width of the slot (19).

4. Intermedullary pin as claimed in one of the claims 1 to 3, characterized in that the cross-sectional profile approximately comprises the form of a three-leaf clover.

5. Intermedullary pin as claimed in one of the claims 1 to 4, characterized in that the inner radius defined by the inner elevations (16) continuously decreases in the slotted area from the distal end towards proximal orientation.

6. Intermedullary pin as claimed in one of the claims 1 to 5, characterized in that the spreading screwbolt (12) comprises an enlargement (26) of the diameter at the end of the shaft, that apertures (27) aligned transversely with the longitudinal axis in the shaft (10) are formed to receive a safety pin and are arranged such, that the spreading pin (12) engages the enlargement (26) of the diameter when the screwbolt is threaded out of the shaft (10).

7. Intermedullary pin as claimed in one of the claims 1 to 6, characterized in that the end of the screwbolt shaft (23) comprises an internal polygonal head to receive the polygonal end of a screw driver.

8. Intermedullary pin as claimed in one of the claims 1 to 7, characterized in that two apertures (28, 29) aligned transversely with the longitudinal axis of the shaft are formed at a small distance to the proximal end of the pin shaft (10), the apertures being adapted to receive a bone screw.

9. Intermedullary pin as claimed in one of the claims 1 to 8, characterized in that in the proximal area of the pin shaft (10) a portion (13) is deflected under a small angle and the wall of the shaft comprises an elongated aperture (29) in the portion (13) in the area wherein the axis of the straight part of the shaft penetrates the wall of the deflected portion (13).

10. Intermedullary pin as claimed in one of the claims 1 to 9, characterized in that two diametrically opposed recesses are formed in the proximal end of the pin shaft (10).

11. Intermedullary pin as claimed in one of the claims 1 to 10, characterized in that an internal thread (30) is formed at a small distance from the proximal end of the shaft.

12. Intermedullary pin as claimed in claim 11, characterized in that at the proximal end of the internal thread (30), an internal cone (31) is formed which is enlarged proximally.

13. Intermedullary pin as claimed in one of the claims 1 to 2, characterized in that a clamp (11) is connectable to the proximal end of the supporting shaft (10), the clamp comprising spider-like arranged bendable arms (34) which are somewhat bent downwardly.

14. Intermedullary pin as claimed in claim 13, characterized in that the arms (34) are equally spaced along the circumferene wherein, however, only in an area of about 200° of the circumference arms (34) are arranged.

15. Intermedullary pin as claimed in claims 13 or 14, characterized in that the rotational position of the clamp (11) is selectable.

16. Intermedullary pin as claimed in one of the claims 13 to 15, characterized in that the clamp (11) comprises an axial conical pin (32) in the center thereof, the pin co-operating with an internal cone (31) of the pin shaft (10), that the pin (32) comprises an axial throughbore (35) for attaching a screwbolt (37), the head thereof (39) abutting against the outer side of the clamp (11) and the shaft (38) thereof co-operating with an internal thread (30) of the supporting shaft (10).

17. Intermedullary pin as claimed in claim 16, characterized in that the head (39) of the attaching screwbolt (37) is rounded and the rounding corresponds with the outer radius of the bent arms (34).

## Revendications

1. Broche d'ostéosynthèse destinée au traitement des fractures humérales, comportant une tige creuse (10) qui, dans la zone proximale, peut être introduite dans le canal médullaire et qui présente, à l'extrémité distale, plusieurs fentes parallèles à l'axe (19), un corps d'expansion (24) qui coopère avec une vis à l'intérieur de la tige (10), qui peut être inséré dans la tige (10) par rotation de la vis et qui élargit radialement l'extrémité fendue, le profil transversal de la tige (10) présentant en direction périphérique, au moins sur le côté intérieur, des bossages (16) et des évidements (17) en alternance, caractérisée en ce qu'un filetage intérieur (25) est formé dans les bossages intérieurs, en dehors de la zone fendue (19, 20), et en ce que le corps d'expansion est constitué par la tête (24) d'une vis d'expansion (12), dont la tige (23) présente une partie filetée (26) qui coopère avec le filetage intérieur (25).

2. Broche d'ostéosynthèse selon la revendication 1, caractérisée en ce que les fentes (19) parallèles à l'axe sont situées dans la zone des évidements (17).

3. Broche d'ostéosynthèse selon la revendication 1 ou 2, caractérisée en ce que les fentes (19) se terminent sur le côté proximal en un trou (20), dont le diamètre est plus grand que la largeur de la fente (19).

4. Broche d'ostéosynthèse selon l'une des revendications 1 à 3, caractérisée en ce que le profil transversal présente à peu près la forme d'un trèfle à trois feuilles.

5. Broche d'ostéosynthèse selon l'une des revendications 1 à 4, caractérisée en ce que le rayon intérieur défini par les bossages intérieurs (16) diminue constamment dans la zone fendue depuis l'extrémité distale vers l'extrémité proximale.

6. Broche d'ostéosynthèse selon l'une des revendications 1 à 5, caractérisée en ce que la vis d'expansion (12) présente un accroisement de diamètre (26) à son extrémité de tige, en ce que des trous orientés transversalement à l'axe longitudinal (27) sont ménagés dans la tige (10) pour recevoir une goupille de sécurité et sont disposés de telle façon que la vis d'expansion (12) vienne en contact par sa partie à diamètre accru (29) avec ladite goupille lorsque la vis est dévissée hors de la tige (10).

7. Broche d'ostéosynthèse selon l'une des revendications 1 à 6, caractérisée en ce que l'extrémité de la tige de vis (23) présente une cavité polygonale intérieure pour recevoir l'extrémité polygonale d'un tournevis.

8. Broche d'ostéosynthèse selon l'une des revendications 1 à 7, caractérisée en ce que deux trous (28, 29) orientés transversalement à l'axe longitudinal de tige sont ménagés à faible distance de l'extrémité proximale de la tige de broche (10) pour recevoir une vis d'ostéosynthèse.

9. Broche d'ostéosynthèse selon l'une des revendications 1 à 8, caractérisée en ce qu'une partie (13) de la zone proximale de la tige de broche (10) est déviée selon un angle faible et en ce que la paroi de tige dans la partie (13) présente un trou allongé (29) dans la zone dans laquelle l'axe de la partie droite de tige rencontre la paroi de la partie déviée (13).

10. Broche d'ostéosynthèse selon l'une des revendications 1 à 9, caractérisée en ce que deux évidements diamétralement opposés sont formés dans l'extrémité proximale de la tige de broche (10).

11. Broche d'ostéosynthèse selon l'une des revendications 1 à 10, caractérisée en ce qu'un filetage intérieur (30) est formé à faible distance de l'extrémité proximale de la tige.

12. Broche d'ostéosynthèse selon la revendication 11, caractérisée en ce qu'un cône intérieur, qui s'élargit vers le côté proximal, est formé sur le côté proximal du filetage intérieur (30).

13. Broche d'ostéosynthèse selon l'une des revendications 1 à 12, caractérisée en ce qu'une bride de fixation (11), qui présente des bras flexibles (34) légèrement recourbés vers le bas et disposés en araignée, peut être reliée à l'extrémité proximale de la tige de broche (10).

14. Broche d'ostéosynthèse selon la revendication 13, caractérisée en ce que les bras (34) sont disposés à des distances périphériques égales, les bras (34) n'étant pourtant répartis que sur une zone périphérique d'environ 200°.

15. Broche d'ostéosynthèse selon la revendication 13 ou 14, caractérisée en ce que la position angulaire de la bride (11) peut être choisie.

16. Broche d'ostéosynthèse selon l'une des revendications 13 à 15, caractérisée en ce que la bride de fixation (11) présente en son centre une tige axiale conique (32) qui coopère avec un cône intérieur (31) de la tige de broche (10), en ce que la tige (32) présente un alésage axial (35) destiné à une vis de fixation (37), dont la tête (39) s'appuie contre le côté extérieur de la bride (11) et dont la tige (38) coopère avec un filetage intérieur (30) de la tige de broche (10).

17. Broche d'ostéosynthèse selon la revendication 16, caractérisée en ce que la tête (39) de la vis de fixation (37) est arrondie et en ce que l'arrondi est adapté au rayon extérieur des bras recourbés (34).

EP 0 226 701 B1

Fig.1

Fig.2

Fig.3

Fig.4

# Fig.5

# Fig.6  Fig.7

# Fig.8

# Fig. 9

34   33   36   11

32   35   34

# Fig. 10

40

39

38   37

41